(19) Europäisches Patentamt / European Patent Office / Office européen des brevets

(11) **EP 4 378 325 A2**

(12) # DEMANDE DE BREVET EUROPEEN

(43) Date de publication:
**05.06.2024 Bulletin 2024/23**

(21) Numéro de dépôt: **24171286.8**

(22) Date de dépôt: **05.01.2018**

(51) Classification Internationale des Brevets (IPC):
***A23L 27/30*** *(2016.01)*

(52) Classification Coopérative des Brevets (CPC):
**A23L 29/30; A23L 27/33; B01D 61/027;
B01D 61/04; C07H 1/06; C07H 3/02;**
B01D 2311/2697

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **05.01.2017 FR 1750104**

(62) Numéro(s) de document de la (des) demande(s)
initiale(s) en application de l'article 76 CBE:
**18700792.7 / 3 565 419**

(71) Demandeur: **ROQUETTE FRERES
62136 Lestrem (FR)**

(72) Inventeurs:
• **BOIT, Baptiste
59253 LA GORGUE (FR)**
• **LACROIX, Geoffrey
59160 LILLE (FR)**

(74) Mandataire: **Plasseraud IP
66, rue de la Chaussée d'Antin
75440 Paris Cedex 09 (FR)**

Remarques:
Cette demande a été déposée le 19-04-2024 comme demande divisionnaire de la demande mentionnée sous le code INID 62.

(54) **SIROPS CRISTALLISABLES DE D-ALLULOSE**

(57) L'invention se rapporte à un sirop de D-allulose comprenant, en plus du D-allulose, une teneur massique en dimère de D-allulose exprimée en masse sèche inférieure à 1,5%. L'invention porte également sur un procédé de fabrication de ce sirop ainsi que sur l'utilisation de ce sirop pour la fabrication de produits alimentaires ou pharmaceutiques.

**EP 4 378 325 A2**

**Description**

**Domaine de l'invention**

L'invention porte sur un sirop de D-allulose dont une des propriétés avantageuses est qu'il est plus aisément cristallisable que les sirops de l'art antérieur. Un autre objet de l'invention porte sur l'utilisation de ce sirop de D-allulose pour la fabrication de produits alimentaires ou pharmaceutiques. Un autre objet de l'invention porte sur un procédé de fabrication de ce sirop de D-allulose.

**Art antérieur**

Le D-allulose (ou D-psicose) est un sucre rare présentant un pouvoir sucrant égal à 70% de celui du saccharose. Contrairement à ce dernier, le D-allulose ne provoque pas de prise de poids car il n'est pas métabolisé par les humains. Il présente une très faible valeur calorique (0,2 kcal par gramme) et il empêche ainsi la prise de masse graisseuse. De plus, des études ont montré que le D-allulose est non cariogène, voire anti-cariogène. C'est ainsi que ces propriétés ont généré récemment un intérêt très important des industries alimentaires et pharmaceutiques.

Le D-allulose est généralement obtenu par voie enzymatique, en faisant réagir une solution aqueuse de D-fructose avec une D-psicose epimerase telle que décrite par exemple dans la demande WO2015/032761 A1 au nom de la Demanderesse. Quelle que soit l'enzyme utilisée, la réaction n'est pas totale et la quantité de fructose transformée en D-allulose après épimérisation est inférieure à 30%.

Ainsi, si l'on souhaite obtenir une composition présentant une teneur massique, exprimée en masse sèche, plus riche en D-allulose, il est nécessaire de réaliser une étape de séparation du D-allulose, ceci afin de l'isoler des autres constituants présents et notamment du fructose. Pour réaliser cette séparation, on réalise très généralement une chromatographie de la composition issue de la réaction d'épimérisation, par exemple par chromatographie continue de type lit mobile simulé, ce qui permet d'isoler une fraction riche en D-allulose.

Le document JP2001354690 A décrit un procédé de purification d'une composition de D-allulose partant d'un mélange de fructose et de D-allulose, ledit procédé comprenant une étape de séparation consistant en une étape de chromatographie continue utilisant une séquence particulière de prélèvements des différents produits du mélange. Une fraction riche en D-allulose (dont la richesse en D-allulose peut atteindre 98%) et une fraction riche en fructose sont récupérées. Le rendement de récupération dans la fraction riche en D-allulose est de 96%.

A l'issue des étapes de séparation citées ci-dessus, des compositions liquides riches en D-allulose sont obtenues. C'est ainsi que ces compositions liquides, généralement appelées sirops, sont utilisées pour la fabrication de produits alimentaires ou pharmaceutiques. A titre d'exemple, la demande WO 2015/094342 également au nom de la Demanderesse décrit la fabrication de produits alimentaires solides comprenant un sirop de D-allulose, comprenant de 50 à 98% de D-allulose et une protéine native. C'est principalement sous cette forme de sirops que différentes sociétés ont annoncé la commercialisation de D-allulose à ce jour.

Le document WO 2016/135458 décrit quant à lui des sirops comprenant, par rapport à sa masse sèche, au moins 80% d'allulose et en étudie leur stabilité au cours du temps. Aucun protocole de fabrication de ce sirop n'est décrit. La composition des sirops est analysée par chromatographie liquide haute performance, qui est présentée comme la méthode utilisée de manière standard d'analyse de ce type de sirops. Les sirops décrits dans cette demande sont présentés comme présentant un caractère faiblement cristallisable.

Or, pour certaines applications, il peut y avoir un intérêt à utiliser un sirop de D-allulose susceptible de cristalliser plus facilement. C'est notamment le cas pour la fabrication de biscuits où l'utilisation d'un sirop cristallisable permet la fabrication de biscuits plus croustillants. Un autre exemple porte sur la fabrication de pâtes à mâcher à texture courte où l'utilisation d'un sirop cristallisable est nécessaire afin d'obtenir la texture en bouche courte caractéristique de ces pâtes à mâcher. Un sirop plus cristallisable permet également d'augmenter la dureté des chewing-gums et ainsi d'améliorer la texture en bouche au moment de la mâche ou d'augmenter la dureté des caramels à texture longue.

C'est en menant de nombreuses recherches que la Demanderesse a pu constater que, dans un procédé de fabrication de sirops de D-allulose, des impuretés particulières se forment au cours du procédé. Celles-ci n'ont, à la meilleure connaissance de la Demanderesse, jamais été reportées dans la littérature. Elles ont pu être identifiées par la Demanderesse, en utilisant une technique particulière de chromatographie phase gaz, comme étant des dimères de D-allulose. La Demanderesse a également pu montrer que ces dimères, contrairement à d'autres impuretés telles que le glucose ou le fructose, ont un effet anti-cristallisant très important. Or, comme ces dimères de D-allulose se forment lors du procédé, leur présence dans le sirop de D-allulose est systématique, et ceci même si toutes les précautions ont été prises lors de sa fabrication. Ceci rend le sirop faiblement cristallisable et ainsi limite leurs usages, notamment dans les applications sus-mentionnées.

Dépassant ce constat, la Demanderesse a en outre poursuivi ses efforts afin de fournir de nouveaux sirops présentant un caractère cristallisable plus important que ceux de l'art antérieur. Pour ce faire, elle a mis au point un procédé particulier lui permettant d'éliminer en grandes partie ces impuretés des sirops de D-allulose.

## Résumé de l'invention

L'invention a ainsi pour objet un sirop de D-allulose comprenant, en plus du D-allulose, une teneur massique en dimère de D-allulose, déterminée par chromatographie phase gaz (CPG), inférieure à 1,5%.

Ce sirop présente l'avantage d'être plus cristallisable que les sirops de l'art antérieur de mêmes matière sèche et teneur en D-allulose. Ceci permet des utilisations avantageuses, notamment pour la fabrication de biscuits ou de pâtes à mâcher.

Un autre objet de l'invention sur un procédé de fabrication du sirop de l'invention qui comprend :

- une étape de fourniture d'une composition aqueuse de D-allulose comprenant des dimères de D-allulose ;
- une étape de nanofiltration de ladite composition de D-allulose pour fournir un rétentat et un perméat ;
- une étape de récupération du perméat de nanofiltration ;
- une étape de concentration de ce perméat pour fournir le sirop de D-allulose.

L'utilisation d'une étape de nanofiltration permet de récupérer un perméat quasiment exempt voire exempt de dimère de D-allulose et d'ainsi obtenir, après l'étape de concentration le sirop de l'invention.

Comme évoqué ci-dessus, la Demanderesse a pu constater que, systématiquement, lors de la fabrication de sirops de D-allulose, des impuretés particulières se forment au cours du procédé. Celles-ci n'ont, à la meilleure connaissance de la Demanderesse, jamais été reportées dans la littérature. Ceci s'explique par le fait que, par la technique de chromatographie liquide haute performance classiquement utilisée pour mesurer la pureté du D-allulose, ces impuretés ne sont pas détectées sur les chromatogrammes (voir Figures 4 et 5). C'est en utilisant une technique de chromatographie en phase gaz que la Demanderesse a pu détecter leur présence (voir Figures 6 et 7).

En ce qui concerne le document WO2015/094342, ce document utilise des sirops comprenant du D-allulose. Toutefois, aucune méthode de préparation de ce sirop n'est indiquée. Or, comme il apparaît dans la suite de la description, le choix des conditions des étapes de préparation et notamment de concentration ont un impact capital sur la quantité de dimères de D-allulose formés qui n'avaient jusqu'ici jamais été reportées. Le document ne décrit donc pas le sirop de l'invention. Il en est de même dans le document WO2016/135458 déjà évoqué ou encore le document WO2015/032761 qui n'exemplifie même pas un tel sirop.

En ce qui concerne le document JP2001354690 A cité précédemment, il décrit la séparation du fructose et de l'allulose par chromatographie d'une composition comprenant ces deux constituants. Toutefois, il ne décrit pas la préparation d'un sirop de D-allulose. Or, comme le choix des conditions des étapes de préparation de ce sirop et notamment de concentration ont un impact capital sur la quantité de dimères de D-allulose, ce document ne décrit donc pas le sirop de l'invention.

Les documents WO2011119004 A2, WO 2016/064087, CN 104447888 A, CN 103333935 A décrivent la fabrication de compositions cristallines comprenant du D-allulose mais ne décrivent pas la fabrication de sirops de D-allulose. L'invention porte également sur un sirop de D-allulose susceptible d'être obtenu par le procédé de l'invention.

## Brève description des Figures

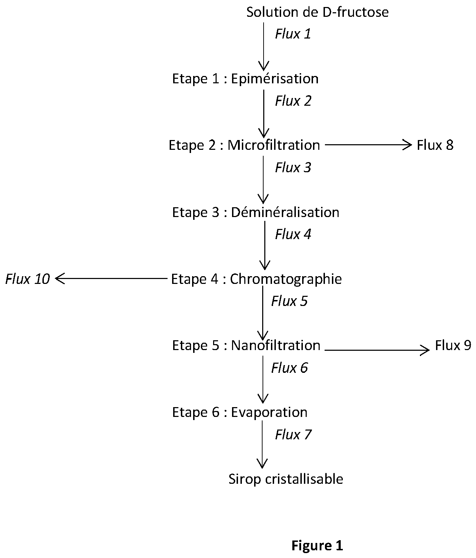

Figure 1 : La Figure 1 représente le circuit de production d'un sirop d'allulose cristallisable.

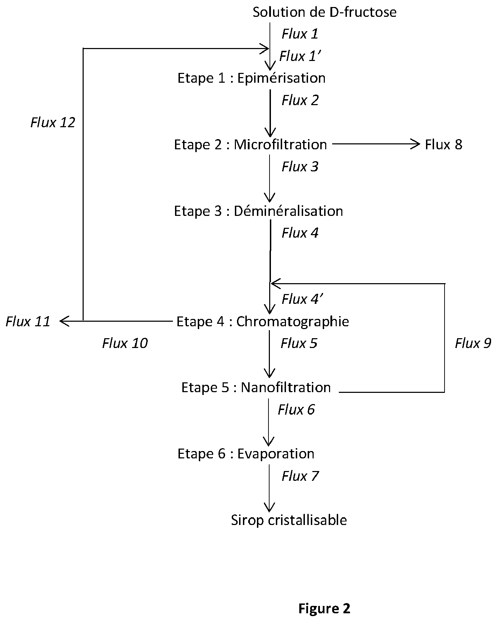

Figure 2 : La Figure 2 représente un circuit de production d'un sirop d'allulose cristallisable avec des boucles de recyclage.

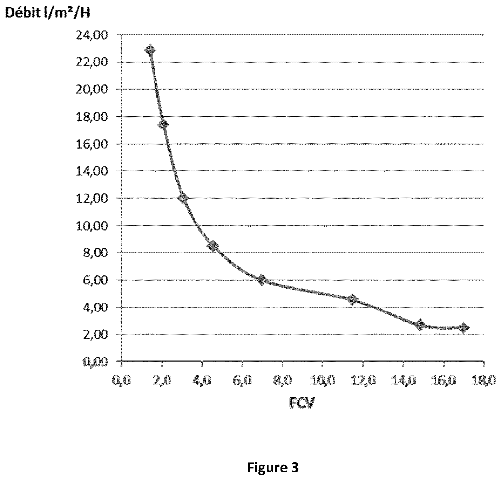

Figure 3 : La Figure 3 représente la courbe de perméation relative à l'étape de nanofiltration, c'est-à-dire le débit en fonction du facteur de concentration volumique.

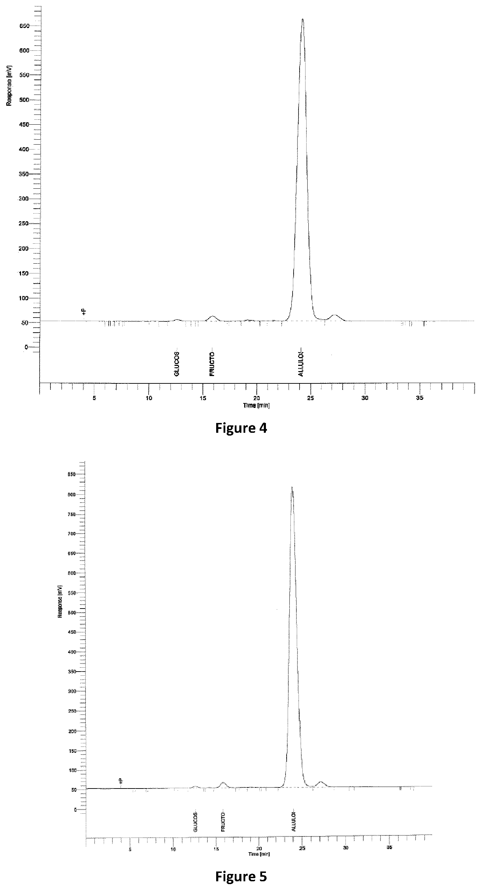

Figure 4 : La Figure 4 représente un chromatogramme HPLC d'une composition riche en D-allulose prélevée dans le procédé de l'invention, avant nanofiltration.

Figure 5 : La Figure 5 représente un chromatogramme HPLC d'un perméat prélevé dans le procédé de l'invention, c'est-à-dire après nanofiltration.

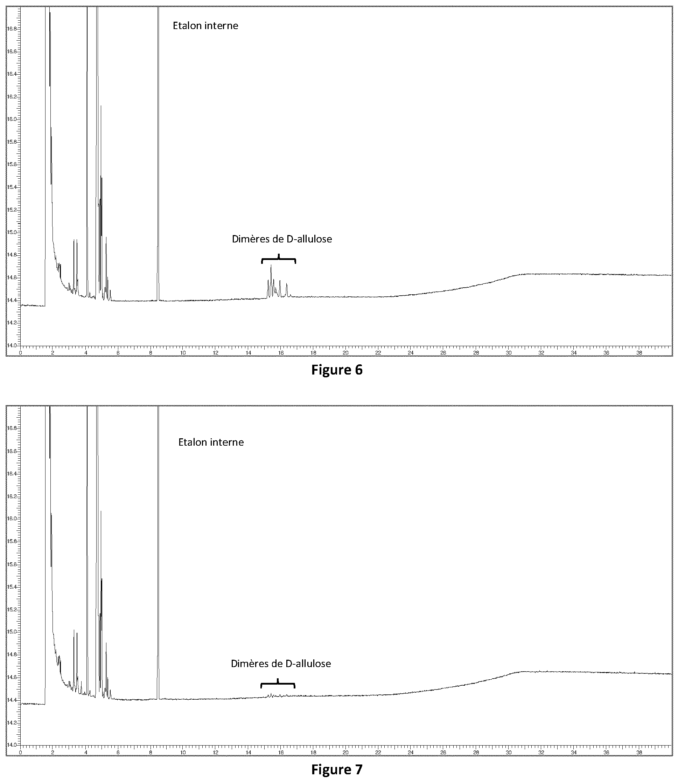

Figure 6 : La Figure 6 représente un chromatogramme CPG, dans la zone caractéristique des dimères, d'une composition riche en D-allulose prélevée dans le procédé de l'invention, avant nanofiltration.

Figure 7 : La Figure 7 représente un chromatogramme CPG, dans la zone caractéristique des dimères, d'un perméat prélevé dans le procédé de l'invention, c'est-à-dire après nanofiltration.

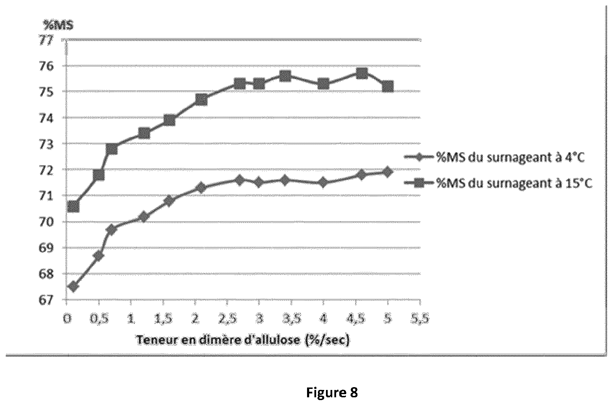

Figure 8 : La Figure 8 représente la matière sèche du surnageant d'un sirop de D-allulose après 1 mois de stockage à 4 et 15°C en fonction de la teneur en dimères de D-allulose.

**Description détaillée de l'invention**

Le sirop de D-allulose de l'invention est une solution aqueuse qui est appauvrie en dimères de D-allulose. Par « composition aqueuse » ou « solution aqueuse », on entend généralement une composition ou une solution dont le solvant est constitué essentiellement d'eau. Par « dimère de D-allulose », on entend un composé comprenant un D-allulose condensé avec au moins un second monosaccharide identique ou différent. Ces dimères sont par exemple des dimères de type D-allulose-D-allulose.

Comme décrit précédemment, le fait que la quantité en dimères de D-allulose comprise dans le sirop soit appauvrie, i.e. que sa teneur massique exprimée en masse sèche soit selon l'invention inférieure à 1,5%, a permis d'obtenir un sirop plus cristallisable.

Ces dimères ont pu être détectés par CPG et n'ont pas pu être détectés lors de l'analyse HPLC, comme le démontrent les Figures 4 à 7. Il en découle que les quantités massiques des différents constituants, exprimées en masse sèche, sont dans la présente demande systématiquement déterminées par CPG. Pour déterminer les quantités de chacune des espèces dans la composition, l'échantillon subit généralement une étape de traitement afin de transformer les différentes espèces présentes en dérivés méthoximés triméthylsilylés. Les quantités massiques de chacune des espèces sont exprimées dans la présente Demande, sauf mention contraire, par rapport à la masse sèche totale.

Les quantités de glucose, de fructose et d'allulose peuvent être déterminées dans un chromatographe en phase gazeuse équipé d'un injecteur chauffé à 300°C, d'un détecteur à ionisation de flamme (FID) chauffé à 300°C et équipé d'une colonne DB1 capillaire de 40 mètres, présentant un diamètre interne de 0,18 mm et une épaisseur de film de 0,4 $\mu$m, la température de la colonne étant programmée de manière suivante : de 200°C jusqu'à 260°C à raison de 3°C/min, puis de 260 °C jusqu'à 300°C à 15°C/min, maintien à 300°C pendant 5 min.

Par quantité de dimères de D-allulose, on entend la différence entre la quantité totale de dimères dans un échantillon, déterminée par CPG, et la quantité des dimères connus éventuellement présents, qui sont des dimères glucose-glucose tels que le maltose et l'isomaltose. Toutefois, la quantité de ces dimères glucose-glucose est généralement très faible, voire inexistante. Par exemple, dans le sirop de l'invention, la quantité massique de dimères glucose-glucose est généralement inférieure à 0,2%, souvent inférieure à 0,1%.

La quantité éventuelle de dimères glucose-glucose peut être déterminée dans les mêmes conditions que celles décrites précédemment pour le glucose, le fructose et le D-allulose :

- en réalisant une hydrolyse des dimères glucose-glucose de l'échantillon ;
- en déterminant la quantité de glucose total dans le même chromatographe et dans les mêmes conditions, le dit glucose total comprenant le glucose initial dit libre et le glucose issu de l'hydrolyse des dimères glucose-glucose ;
- en soustrayant, à cette quantité de glucose total, la quantité de glucose initial de l'échantillon.

La quantité totale de dimères peut quant à elle être déterminée dans un chromatographe en phase gazeuse dans les mêmes conditions que décrites précédemment, à la différence que la colonne utilisée est une colonne DB1 capillaire de 30 mètres, présentant un diamètre interne de 0,32 mm et une épaisseur de film de 0,25 $\mu$m et la température de la colonne est programmée de manière suivante : de 200°C jusqu'à 280°C à raison de 5°C/min, maintien à 280°C pendant 6 min, puis de 280 °C jusqu'à 320°C à 5°C/min, maintien à 320°C pendant 5 min.

La méthode est décrite plus en détail dans la partie Exemples.

Le sirop de D-allulose comprend, en plus du D-allulose, une teneur massique en dimère de D-allulose, déterminée par chromatographie phase gaz (CPG), inférieure à 1,5%. Le sirop de D-allulose de l'invention peut présenter une teneur massique en dimère de D-allulose, allant de 0,1 à 1,4%, avantageusement allant de 0,2 à 1,3%, préférentiellement allant de 0,3 à 1,2%.

Le sirop de D-allulose présente un caractère encore plus cristallisable lorsque sa teneur en D-allulose exprimée en masse sèche est supérieure ou égale à 75%. Il peut présenter une teneur en D-allulose exprimée en masse sèche supérieure ou égale à 80%, par exemple supérieure ou égale à 85%, notamment supérieure ou égale à 90%. Plus la teneur de D-allulose exprimée en masse sèche est importante, plus le sirop est cristallisable.

Le sirop de D-allulose peut avantageusement comprendre, par rapport à sa masse sèche :

- de 75 à 99% de D-allulose ;
- de 0 à 25% de D-fructose ;
- de 0 à 10% de glucose ;
- de 0 à 1,5 % (borne exclue) de dimère de D-allulose, par exemple allant de 0,1 à 1,4%, avantageusement allant de 0,2 à 1,3%, préférentiellement allant de 0,3 à 1,2%.

Le sirop de D-allulose peut présenter une matière sèche supérieure à 50%, par exemple allant de 65 à 85%, notamment allant de 70 à 83%, par exemple de 75 à 82%. Plus la matière sèche est importante, plus le sirop est aisément cristallisable. Toutefois, lorsque la matière sèche est élevée, la viscosité du sirop peut augmenter, ce qui peut conduire à des difficultés dans sa manipulation.

Un sirop de D-allulose est classiquement obtenu par un procédé comprenant :

- une étape de fourniture d'une composition aqueuse comprenant du D-allulose ;
- une étape de concentration de ladite composition aqueuse pour former le sirop de D-allulose.

Le sirop de l'invention peut être réalisé selon un procédé décrit en détail ci-après qui comprend, préalablement à l'étape de concentration, une étape de nanofiltration.

Cette étape de nanofiltration permet de limiter la quantité de dimères de D-allulose dans le sirop de l'invention. Cette étape de nanofiltration a lieu avant l'étape de concentration de la composition riche en D-allulose. Cette étape permet donc la fourniture d'un sirop de D-allulose dont la teneur en dimères de D-allulose est plus faible que celle obtenue à partir d'un même procédé n'utilisant pas cette étape de nanofiltration.

Dans l'étape de nanofiltration, qui est essentielle au procédé de l'invention, deux fractions sont formées lorsque l'on soumet une composition de D-allulose à nanofiltrer :

- un perméat, qui est appauvri en dimères de D-allulose ;
- ainsi qu'un rétentat, qui est enrichi en dimères de D-allulose.

Dans la Figure 1 qui représente un circuit de production de sirop de l'invention, le Flux 6 représente le perméat et le Flux 9 représente le rétentat. Pour des raisons illustratives mais non limitatives, sauf indication contraire, les Flux indiqués dans la suite de la description se réfèrent aux flux du circuit de production de cette Figure 1.

Le perméat de nanofiltration est un intermédiaire permettant la fabrication de cette solution mère.

Les termes « appauvri en dimères de D-allulose» et « enrichi en dimères de D-allulose » sont évidemment relatifs par rapport à la teneur en oligomères de D-allulose de la composition à nanofiltrer.

Le perméat de nanofiltration est un intermédiaire permettant la fabrication du sirop de D-allulose de l'invention.

Un objet de l'invention porte donc sur un procédé de fabrication de sirop qui comprend :

- une étape de fourniture d'une composition aqueuse de D-allulose comprenant des dimères de D-allulose ;
- une étape de nanofiltration de ladite composition de D-allulose pour fournir un rétentat et un perméat ;
- une étape de récupération du perméat de nanofiltration ;
- une étape de concentration de ce perméat pour fournir le sirop de D-allulose de l'invention.

Pour réaliser l'étape de nanofiltration utile à l'invention, la composition à nanofiltrer est passée sur une membrane de nanofiltration. Elle présente généralement une matière sèche allant de 5 à 15%.

La température de cette composition à nanofiltrer peut aller de 10 à 80 °C, généralement de 15 à 50°C, souvent aux alentours de 20 °C.

L'Homme du métier saura choisir la membrane utile à cette séparation. Cette membrane de nanofiltration peut avoir un seuil de coupure inférieur à 300 Da, de préférence allant de 150 à 250 Da. Idéalement, la membrane a un taux de réjection de MgSO4 d'au moins 98%. Il peut notamment s'agir d'une membrane de type Dairy DK ou Duracon NF1 fabriquées par GE®.

La pression appliquée à la membrane peut également varier largement et peut aller de 1 à 50 bars, de préférence de 5 à 40 bars, tout préférentiellement de 15 à 35 bars.

Cette étape de nanofiltration peut être accompagnée d'une phase de diafiltration.

De préférence, le facteur de concentration volumique (FCV) de la nanofiltration va de 2 à 20. Ce facteur de concentration volumique est aisément réglé par l'Homme du métier.

Cette étape de nanofiltration peut être réalisée en continu.

A l'issue de cette étape de nanofiltration, le perméat récupéré peut comprendre, par rapport à sa masse sèche, de 0 à 1,2% de dimères de D-allulose, par exemple de 0,05 à 1,0%, notamment de 0,1 à 0,5%.

Il va sans dire que le procédé selon l'invention peut comprendre d'autres étapes, telles que les autres étapes figurant dans le procédé classique décrit précédemment et qui vont être décrites en détail par la suite. Le procédé selon l'invention peut également comprendre des étapes additionnelles de purification et également des étapes de dilution ou de concentration intermédiaires en vue de régler la matière sèche et ainsi réaliser dans les meilleures conditions les différentes étapes du procédé de l'invention. L'ensemble de ces étapes peuvent être réalisées en continu.

Le sirop de l'invention présente généralement une matière sèche supérieure ou égale à 50%. Pour augmenter la matière sèche (le perméat a une matière sèche plus faible que 50%), il est nécessaire de réaliser une étape de concentration, pendant laquelle la teneur en dimères de D-allulose peut augmenter. La formation de dimères de D-allulose se produisant également lors de cette étape de concentration, il est préférable de sélectionner des conditions permettant de limiter les quantités formées en ces dimères. L'étape de concentration est ainsi généralement réalisée sous vide, par exemple à une pression de 5 à 100 mbars, de préférence allant de 20 à 70 mbars. Ce vide permet de diminuer la température nécessaire à l'évaporation et de réduire la durée de cette étape de concentration. Elle peut être réalisée à une température allant de 30 à 80 °C, avantageusement de 34 à 70°C, préférentiellement de 37 à 50°C. Cette étape de concentration peut être réalisée dans un évaporateur simple étage, un évaporateur multiple étages, par exemple un évaporateur double étages. A l'issue de l'étape de concentration, le sirop de D-allulose de l'invention peut être obtenu. Il comprend une teneur en dimères de D-allulose inférieure à 1,5%. Généralement, le sirop de l'invention comprend de 0 à 1,2% de dimères de D-allulose, par exemple de 0,1 à 1,2%, notamment de 0,2 à 1,0% ou encore de 0,3 à 0,8%.

Le procédé de l'invention comprend en outre une étape de fourniture d'une composition aqueuse de D-allulose comprenant des dimères de D-allulose. Les teneurs massiques des différents constituants du sirop (et notamment le D-allulose, le D-fructose et le glucose éventuels) sont principalement déterminées par les teneurs en chacun de ces constituants compris dans la composition aqueuse de D-allulose fournie. Par exemple, si la composition aqueuse de D-allulose fournie présente une teneur élevée en D-allulose, le perméat et le sirop obtenu à partir de ce perméat présentent également une teneur élevée en D-allulose.

Un procédé classique de fabrication d'une composition de D-allulose comprenant des dimères de D-allulose comprend :

- une étape de fourniture d'une solution de D-fructose ;
- une étape d'épimérisation de ladite solution pour former une composition de D-allulose, comprenant du D-fructose et du D-allulose ;
- éventuellement une étape de chromatographie pour enrichir en D-allulose la composition de D-allulose ;
- une étape de concentration de la composition, éventuellement enrichie, en D-allulose.

Ainsi, selon le procédé de l'invention, pour fournir la composition de D-allulose, il peut être réalisé une étape de chromatographie d'une composition comprenant du D-allulose et du D-fructose. Dans ce cas, cette composition comprenant du D-allulose et du D-fructose est avantageusement obtenue par épimérisation d'une solution de D-fructose.

La composition de D-allulose obtenue après l'étape de chromatographie, qui présente une teneur en D-allulose supérieure à celle de la composition obtenue à l'issue de l'étape d'épimérisation, comprend des dimères de D-allulose. En plus de cette composition de D-allulose, est également formée, lors de cette étape de chromatographie, une composition riche en D-fructose ou « raffinat ».

La composition de D-fructose fournie (Flux 1) pour réaliser l'étape d'épimérisation peut être un sirop de D-fructose, qui peut être obtenu par dissolution de cristaux de D-fructose dans de l'eau ou un sirop de glucose/D-fructose. Préférentiellement, cette composition comprend un sirop de glucose/D-fructose qui comprend au moins 90% en poids sec de D-fructose, préférentiellement au moins 94% de D-fructose.

Dans un mode représenté dans la Figure 2, la composition de D-fructose fournie pour réaliser l'étape ultérieure d'épimérisation est un mélange (Flux 1') de ce sirop de D-fructose avec au moins une fraction recyclée qui peut être le raffinat (la totalité du raffinat ou une partie) (Flux 10 ou 12), cette fraction recyclée pouvant comprendre une quantité de D-allulose supérieure.

La composition de D-fructose soumise à l'étape d'épimérisation peut comprendre :

- de 0 à 10% de D-allulose ;
- de 70 à 100% de D-fructose ;
- de 0 à 10% de glucose ;
- de 0 à 15% de dimère de D-allulose.

L'étape d'épimérisation est réalisée à partir de la composition de D-fructose fournie précédemment, éventuellement après réglage de la matière sèche. Cette étape est généralement réalisée à une matière sèche allant de 30 à 60%, souvent de 45 à 55%. On introduit dans cette composition une enzyme de type D-psicose epimerase ou une composition comprenant cette enzyme. La composition comprenant cette enzyme peut être un lyophilisat d'un microorganisme hôte synthétisant la D-psicose epimerase, celui-ci pouvant être le *Bacillus subtilis,* notamment celui décrit dans la demande WO2015/032761 A1. Le pH est réglé selon l'enzyme utilisée, par exemple à un pH allant de 5,5 à 8,5. La réaction peut être réalisée en chauffant à une température allant de 40 à 70°C, souvent de 45 à 60 °C. La réaction peut durer de 0,1 à 100 heures, par exemple de 0,2 à 60 heures. Cette réaction peut par exemple se faire sur une colonne enzymatique, ce qui présente l'avantage de travailler également en continu sur cette étape. Il est également possible pour fonctionner en continu de travailler séquentiellement avec plusieurs réacteurs. Pour réaliser cette étape d'épimérisation, on peut notamment utiliser l'enseignement du document WO 2015/032761 A1.

A l'issue de la réaction est formée une composition comprenant du D-fructose et du D-allulose, généralement selon un ratio massique D-fructose / D-allulose allant de 85/15 à 55/45, souvent selon un ratio massique D-fructose / D-allulose allant de 80/20 à 60/40. Ce ratio dépend des paramètres d'épimérisation utilisés et, bien évidemment, de la quantité de D-allulose et de D-fructose dans la composition de D-fructose fournie dans l'étape d'épimérisation ; la quantité de D-allulose dans cette composition peut être notamment plus importante en cas de recyclage.

A l'issue de cette étape d'épimérisation, si nécessaire, une étape de filtration peut être réalisée pour récupérer les débris cellulaires éventuellement présents, notamment lorsqu'un lyophilisat d'un microorganisme hôte est utilisé. Cette étape peut consister en une étape de microfiltration. La composition microfiltrée correspond au Flux 3 et les débris cellulaires sont récupérés dans le Flux 8.

Dans le procédé de l'invention, des étapes de purifications additionnelles peuvent également être réalisées. On réalise généralement, avant l'étape de chromatographie, une étape de déminéralisation de la composition comprenant du D-fructose et du D-allulose (Flux 3) qui peut être réalisée par passage sur une ou plusieurs résines échangeuses d'ions cationiques (par exemple une résine cationique de type Dowex 88), anioniques (par exemple une résine anionique de type Dowex 66) et un mélange cationique-anionique. Dans la Figure 3, cette composition correspond au Flux 4. La composition comprenant du D-fructose et du D-allulose obtenue est alors déminéralisée et présente généralement une résistivité supérieure à 100 k$\Omega$.cm$^{-1}$. On peut également réaliser, avant cette étape de déminéralisation, une étape de décoloration de la composition comprenant du D-fructose et du D-allulose, par exemple en passant sur une colonne comprenant du charbon actif.

La composition comprenant du D-fructose et du D-allulose (Flux 4) peut ensuite être soumise à une étape de chromatographie pour fournir au moins une composition riche en D-allulose et une composition riche en D-fructose. Dans un mode préféré qui sera explicité en détail plus loin dans la description, la composition comprenant du D-fructose et du D-allulose soumise à l'étape de chromatographie est un mélange (Flux 4') de la composition issue de l'étape d'épimérisation (Flux 4) et d'au moins une fraction recyclée, cette fraction recyclée pouvant comprendre une quantité de D-allulose supérieure.

La composition soumise à l'étape de chromatographie peut comprendre, par rapport à sa masse sèche :

- de 22 à 45% de D-allulose, généralement de 23 à 37% ;
- de 45 à 75% de D-fructose, généralement de 46 à 70% ;
- de 0 à 10% de glucose ;
- de 2 à 10% de dimère de D-allulose.

Pour réaliser cette étape de chromatographie, on peut utiliser tout type de chromatographie continue, notamment de type chromatographie à lit mobile simulé *(Simulated Moving Bed SMB)*, de type *Improved Simulated Moving Bed (ISMB)*, de type *Divide Improved Simulated Moving Bed (DISMB), de type Sequential Simulated Moving Bed (SSMB)* ou de type *Nippon Mitsubishi Chromatography Improved (NMCI)*. On utilise généralement de l'eau comme éluant. La chromatographie peut être équipée de plusieurs colonnes en série par exemple de 4 à 8 colonnes. Les colonnes comprennent de la résine échangeuse d'ions, par exemple une résine cationique échangeuse d'ions calcium. La matière sèche de la composition comprenant du D-fructose et du D-allulose peut aller de 40 à 70%, généralement est d'environ 50%. La température de la composition lors de la chromatographie va généralement de 40 à 80°C, de préférence de 55 à 65°C. Cette chromatographie dure le temps d'obtenir une séparation satisfaisante et peut durer plusieurs heures.

On obtient à l'issue de cette étape une composition riche en D-allulose (Flux 5) qui peut comprendre, par rapport à sa matière sèche, au moins 80% de D-allulose, avantageusement au moins 90% de D-allulose. Cette composition riche en D-allulose peut présenter une matière sèche allant de 5 à 15%. On obtient également à l'issue de cette étape un raffinat (Flux 10), qui comprend généralement par rapport à sa matière sèche au moins 75% de D-fructose, souvent au moins 80% de D-fructose. Ce raffinat présente généralement une matière sèche allant de 15 à 30% environ.

Le procédé selon l'invention peut comprendre une étape de recyclage d'au moins une partie du rétentat de nanofiltration (Flux 9 de la Figure 2) et/ou du raffinat (Flux 10 de la Figure 2).

Ces recyclages sont susceptibles d'augmenter la quantité de dimères de D-allulose dans les différentes compositions antérieures à l'étape de nanofiltration.

La composition riche en D-allulose obtenue à l'issue de la chromatographie (Flux 5) peut ainsi comprendre, par rapport à sa masse sèche :

- de 80 à 98% de D-allulose ;
- de 0 à 20% de D-fructose ;
- de 0 à 10% de glucose ;
- de 0,5 à 5% de dimère de D-allulose.

Le raffinat (Flux 10) peut comprendre quant à lui, par rapport à sa masse sèche :

- de 1 à 10% de D-allulose ;
- de 70 à 99% de D-fructose ;
- de 0 à 10% de glucose ;
- de 1,3 à 20% de dimère de D-allulose.

Les quantités des différents constituants du sirop de D-allulose de l'invention peuvent être aisément réglées par l'Homme du métier en choisissant la composition de la solution de D-fructose utilisée, les paramètres de l'étape d'épimérisation ainsi que les paramètres de l'étape de chromatographie. Leur choix permet de fournir la composition aqueuse de D-allulose comprenant des dimères de D-allulose utile à l'invention et peut déterminer ainsi les teneurs en D-allulose, D-fructose et glucose finales dans le sirop de l'invention. Il est possible d'enrichir en certains constituants par exemple en ajoutant au perméat des compositions dont la pureté en ces constituants est plus importante que celle du perméat, sous forme liquide ou solide. Par exemple, on peut ajouter des cristaux de D-fructose pour augmenter la teneur en D-fructose du sirop.

Le sirop de l'invention peut être avantageusement utilisé pour la fabrication de produits alimentaires ou pharmaceutiques. Il peut être utilisé dans les applications connues de l'allulose et, de manière générale, des édulcorants. Parmi les applications pouvant utiliser de manière avantageuse le caractère plus cristallisable du sirop de D-allulose selon la présente invention peuvent être citées les chewing-gums sous forme de tablettes ou dragées, les caramels à texture longue, les bonbons et les comprimés à sucer, les biscuits, les cookies, les muffins, les gâteaux, les gâteaux à base de gélatine, les pâtes à mâcher notamment les pâtes à mâcher à texture courte.

L'invention va maintenant être illustrée dans la partie Exemples ci-dessous. Il est précisé que ces Exemples ne sont pas limitatifs de la présente invention.

## Exemples

### *Méthodes analytiques*

Chromatographie en phase gazeuse

Le chromatographe en phase gazeuse utilisé est de type Varian 3800 et est équipé de :

- Un injecteur split-splitless (avec ou sans diviseurs);

- Un détecteur à ionisation de flamme (FID) ;

- Un système informatique permettant de traiter le signal du détecteur ;

- Un échantillonneur automatique (type 8400).

Les différentes quantités sont déterminées par chromatographie en phase gazeuse sous forme de dérivés méthoximés triméthylsilylés, puis quantifiés par la méthode de l'étalonnage interne.

### *Détermination des teneurs en D-allulose, D-fructose et glucose*

Les coefficients de réponse appliqués sont de 1,25 pour le D-allulose et le D-fructose et de 1,23 pour le glucose. Les autres monosaccharides n'ont pas été détectés.

### *Préparation de l'échantillon*

Dans une boîte à tare, peser 100 à 300 mg de l'échantillon à tester + 10 ml solution étalon interne constituée de méthyl $\alpha$-D-glucopyranoside à 0,3mg/ml dans la pyridine. Dans un godet de 2 ml, prélever 0,5 ml de la boîte à tare et évaporer à sec sous courant d'azote. Ajouter 20 mg de chlorhydrate de méthoxylamine et 1 ml de pyridine. Boucher et laisser dans le système d'incubation de type Reacti-therm ® à 70°C pendant 40 min. Ajouter 0,5 ml de N, O Bis (triméthylsilyl) trifluoroacétamide (BSTFA). Chauffer 30 min à 70 °C.

*Conditions chromatographiques*

Colonne : DB1 capillaire 40 mètres, diamètre interne 0,18 mm, épaisseur du film 0,4$\mu$m, constituée à 100 % de diméthylpolysiloxane, apolaire (J&W Scientific réf. : 121-1043)
Température de colonne : 100°C programmation jusqu'à 260°C à raison de 3°C/min, puis jusqu'à 300°C à 15°C/min, maintenir 5 min à 300°C.
Température injecteur : 300°C
Température détecteur : 300°C (Range 10$^{-12}$)
Pression : 40 psi (débit constant)
Gaz vecteur : Hélium
Mode d'injection : Split (Débit de split : 100 ml/min)
Volume injecté : 1,0$\mu$l

Le D-allulose, le D-fructose et le glucose ont été détectés dans cet ordre. Le D-allulose, qui était inconnu, a un temps de rétention dans ces conditions compris entre 39,5 et 40 minutes.

*Détermination des teneurs en dimères de D-allulose et en dimères glucose-glucose*

Les coefficients de réponse appliqués sont de 1,15 pour les dimères de D-allulose et le maltose, et de 1,08 pour l'isomaltose. Les autres dimères de glucose n'ont pas été détectés.

*Préparation de l'échantillon :*

Dans une boîte à tare, peser 100 à 300 mg de l'échantilllon à tester + 10 ml solution étalon interne constituée de Phényl beta-D -glucopyranoside à 0,3 mg/ml dans la pyridine.
Dans un godet de 2 ml, prélever 0,5 ml de la boîte à tare et évaporer à sec sous courant d'azote.
Reprendre par 0,5 ml de la solution de chlorhydrate d'hydroxylamine à 40g/l dans la pyridine, boucher, agiter et laisser 40 min à 70 °C.
Ajouter 0,4 ml de BSTFA et 0,1 ml de N-Trimethylsilylimidazole (TSIM). Chauffer 30 min à 70°C.

*Conditions chromatographiques*

Colonne : DB1 capillaire 30 mètres, diamètre interne 0,32 mm, épaisseur du film 0,25$\mu$m (J&W Scientific réf. : 123-1032)
Température de colonne : 200°C programmation jusqu'à 280°C à raison de 5°C/min (maintenir 6 min), puis jusqu'à 320°C à 5°C/min, maintenir 5 min à 320°C.
Température injecteur : 300°C
Température détecteur : 300°C (Range 10$^{-12}$)
Pression : 14 psi (débit constant)
Gaz vecteur : Hélium
Mode d'injection : Split (Débit de split : 80 ml/min)
Volume injecté : 1,2$\mu$l

Expression des résultats :

La teneur des différents constituants est exprimée en g pour 100 g de produit brut et elle est donnée par l'équation suivante :

$$\% \text{ constituant } i = \frac{Si}{Se} \times \frac{Pe}{P} \times \frac{100}{Ki}$$

Avec :

Si = surface du ou des pics de constituant i
Se = surface du pic d'étalon interne
Pe = Poids d'étalon interne introduit dans le bêcher (en mg)
P = poids d'échantillon pesé (en mg)
Ki = coefficient de réponse du constituant i

Si le pourcentage obtenu (exprimé ici en brut) dépasse 20% pour un des constituants, l'échantillon est dilué et l'analyse CPG recommencée afin d'obtenir une quantité massique inférieure à 20%.

Les quantités massiques exprimées en brut sont ensuite exprimées en sec, en divisant pour la matière sèche de l'échantillon testé.

Les quantités massiques de D-allulose, de D-fructose et de glucose sont déterminées aisément, aucun des pics caractéristiques n'étant co-élués.

Le pic du maltose et des dimères de D-allulose peuvent être co-élués. Il faut noter toutefois que, dans les sirops de l'invention et décrits dans les exemples ci-après, le maltose n'est jamais présent.

Si les pics caractéristiques du maltose ne sont pas détectés, la surface Si des dimères de D-allulose est déterminée par intégration des pics inconnus, entre 10 et 17 minutes. Si les pics caractéristiques du maltose sont détectés (ce qui peut être le cas dans les sirops de l'invention), on détermine les quantités de maltose et on soustrait cette quantité à la quantité totale des dimères.

Pour déterminer la quantité totale de dimères glucose-glucose, on réalise sur un échantillon le protocole suivant :

• Hydrolyse chlorhydrique

Dans un tube à hydrolyse de 15 ml à bouchon vissable en téflon, peser environ précisément de 50 à 500 mg d'échantillon (ajuster la pesée suivant la teneur en sucre attendue), ajouter 2 ml à la pipette à deux traits de la solution d'étalon interne (galactitol à 5 mg/ml dans l'eau osmosée), ajouter 3 ml d'eau et 5 ml de la solution d'HCl 4N.

Boucher hermétiquement, agiter 1 min à l'agitateur Vortex. Placer le tube au bain à sec thermostaté régulé à 100 °C pendant 1 heure en agitant au vortex de temps en temps.

• Déminéralisation et concentration

Après refroidissement, dans un bêcher de 50 ml, déposer la totalité de l'hydrolyse. Ajouter 6 à 8 g d'un mélange à 50/50 de résine anionique AG4 X 4 et AG50 W 8. Laisser sous agitation magnétique pendant 5 minutes. Filtrer sur papier. Récupérer le jus et renouveler l'étape de déminéralisation jusqu'à obtention d'un pH proche de l'eau.

• Préparation de l'échantillon

Dans une boîte à tare, peser 100 à 300 mg de l'échantillon à tester + 10 ml solution étalon interne constituée de méthyl α-D-glucopyranoside à 0,3 mg/ml dans la pyridine. Dans un godet de 2 ml, prélever 0,5 ml de la boîte à tare et évaporer à sec sous courant d'azote. Ajouter 20 mg de chlorhydrate de méthoxylamine et 1 ml de pyridine. Boucher et laisser au Reacti-therm® à 70°C pendant 40 min. Ajouter 0,5 ml de BSTFA. Chauffer 30 min à 70°C.

La quantité de glucose total de la solution (qui comprend le glucose initial dit « libre » et le glucose issu de l'hydrolyse et notamment lié à la présence de maltose et d'isomaltose) est déterminée par analyse CPG du glucose. On en déduit aisément la quantité de maltose et, par différence avec la quantité totale de dimères attribués aux pics entre 10 et 17 minutes, la quantité de dimères de D-allulose.

Exemple 1 : Réalisation d'un procédé industriel continu de fabrication de sirop de D-allulose

L'exemple 1 consiste en une méthode de production en continu de sirop cristallisable de D-allulose. Les étapes du procédé utilisé sont détaillées dans la Figure 1. La composition et le débit des Flux des étapes 1 à 5 sont décrits dans le Tableau 1a.

Etape 1 :

On introduit dans un réacteur batch agité de 14m$^3$ utiles, 17,3 tonnes d'un sirop de D-fructose Fructamyl (Tereos) comprenant 95% de D-fructose à 50% Matière Sèche (MS) (Flux1). Le Flux 1 est maintenu à 55°C. On introduit dans la cuve un lyophilisat de la souche *Bacillus subtilis* hôte de l'enzyme D-Psicose 3 Epimerase détaillée dans le brevet WO2015032761 en quantité suffisante pour avoir 3,3*10$^7$ unités d'activité dans le réacteur. Trois réacteurs sont utilisés séquentiellement de façon à fournir un sirop essentiellement composé de fructose et d'allulose (Flux 2) en continu à un débit de 360kg/h.
Les conditions de la réaction sont les suivantes :

- Température : 55°C

- pH=7

- Temps de réaction 48h

A l'issue de la réaction, le Flux 2 est obtenu comprend une richesse en D-allulose environ égale à 25% et une richesse en D-fructose environ égale à 75%.

Etape 2 :

Le Flux 2 passe à travers une membrane de microfiltration au cours d'une opération batch. On obtient un Flux 3 exempt de débris cellulaires et un rétentat de microfiltration (Flux 8) comprenant les débris issus du lyophilisat de *Bacillus subtilis* qui est purgé du circuit. Les paramètres de microfiltration sont comme suit :

- Pression transmembranaire : 0-3 bar

- Taille de pore : 0,1 μm

- Température : 50°C

- Débit moyen : 15 L/h/m$^2$

- Membrane : Sepro PS35

- Facteur Concentration Volumique : 33

Etape 3 :

Le Flux 3 est déminéralisé sur la résine cationique forte Dowex 88 suivi d'une résine anionique faible Dowex 66 à un débit moyen de 2BV/h. Les bonbonnes sont maintenues à une température de 45°C et la résistivité du Flux 4 à l'issue de la déminéralisation reste supérieure à 100kΩ.cm$^{-1}$ en sortie (Flux 4). Dans le cas contraire la régénération des résines est opérée.

Etape 4 :

Le Flux 4 alimente la chromatographie continue (SCC ARI® équipé de 8 colonnes) du circuit. Le débit moyen d'alimentation est de 348kg/h à 50%MS.
Les paramètres de la chromatographie sont définis comme suit :

- Volume/colonne : 2m$^3$

- Résine : Dowex Monosphere 99Ca/320

- Température : 60°C

- Débit eau/Flux 4 (vol./vol.) : 2,4

- Charge : 0,09 h$^{-1}$

Deux fractions sont extraites : le raffinat (Flux 10), la fraction riche en D-allulose (Flux 5) qui part en direction de l'étape 5. Le Flux 10 est purgé.

Etape 5 :

Le Flux 5 est passé en batch à travers une membrane de nanofiltration. Les paramètres sont comme suit :

- Pression transmembranaire : 30 bar

- Température : 20°C

- Membrane : GE Duracon NF1 8040C35

- Facteur Concentration Volumique FCV : 2,5

Les dimères d'allulose se concentrent dans le rétentat (Flux 9). Ce rétentat est purgé du circuit tandis que le perméat (Flux 6) est récupéré. La figure 6 donne le détail de la perméation des sirops en fonction du FCV.

Etape 6 :

Le Flux 6 passe à travers un évaporateur deux étages dont la pression à l'intérieur est de 50 mbar. Le premier étage est à 38°C et permet d'élever la matière sèche à 35%. Le deuxième étage atteint 77% de matière sèche. On obtient le sirop de D-allulose (Flux 7) à l'issue de cette étape.
Dans le Tableau 1b sont reprises les caractéristiques du sirop de l'invention (Flux 7).

Tableau 1a : Débits et composition des Flux des étapes 1 à 5 de l'Exemple 1

| Etape/Caractéristiques des Flux | Flux | | |
|---|---|---|---|
| Etape 1 | Flux 1 | Flux 2 | - |
| Débit massique (kg/h) | 360 | 360 | |
| Matière Sèche (%) | 50 | 50 | |
| Richesse Fructose (%) | 94,5 | 71,5 | |
| Richesse Glucose (%) | 2 | 2 | |
| Richesse Allulose (%) | 1 | 24 | |
| Richesse Di-Allulose (%) | 1 | 1 | |
| Richesse Autre (%) | 1,5 | 1,5 | |
| | | | |
| Etape 2/Etape 3 | Flux 2 | Flux 3 | Flux 8 |
| Débit massique (kg/h) | 360 | 348 | 12 |
| Matière Sèche (%) | 50 | 50 | 50 |
| | | | |
| Etape 4 | Flux 4 | Flux 10 | Flux 5 |
| Débit massique (kg/h) | 348 | 658 | 424 |
| Matière Sèche (%) | 50 | 20 | 10 |
| Richesse Fructose (%) | 71,5 | 93,2 | 4,4 |
| Richesse Glucose (%) | 2 | 2,5 | 0,4 |

(suite)

| Etape 4 | Flux 4 | Flux 10 | Flux 5 |
|---|---|---|---|
| Richesse Allulose (%) | 23,7 | 1,3 | 93,4 |
| Richesse Di-Allulose (%) | 1,2 | 1,4 | 0,8 |
| Richesse Autre (%) | 1,6 | 1,6 | 1 |
| | | | |
| Etape 5 | Flux 6 | Flux 9 | - |
| Débit massique (kg/h) | 306 | 118 | |
| Matière Sèche (%) | 5 | 23 | |
| Richesse Fructose (%) | 3,5 | 5,0 | |
| Richesse Glucose (%) | 0,3 | 0,4 | |
| Richesse Allulose (%) | 95,8 | 92,2 | |
| Richesse Di-Allulose (%) | 0,1 | 1,1 | |
| Richesse Autre (%) | 0,3 | 1,3 | |

Tableau 1b : Débits et composition du Flux 7

| Etape 6 | Flux 7 | - | - |
|---|---|---|---|
| Débit massique (kg/h) | 19,9 | | |
| Matière Sèche (%) | 77 | | |
| Richesse Fructose (%) | 3,5 | | |
| Richesse Glucose (%) | 0,3 | | |
| Richesse Allulose (%) | 95 | | |
| Richesse Di-Allulose (%) | 0,5 | | |
| Richesse Autre (%) | 0,7 | | |

Dans un même procédé où aucune étape de nanofiltration n'est réalisée, le sirop de D-allulose obtenu est similaire à la différence que la quantité de dimères de D-allulose (Di-Allulose) exprimée en masse sèche est de 1,5%.

**Exemple 2 : Evaluation du caractère cristallisable de différents sirops de D-allulose**

L'exemple 2 consiste à préparer différents sirops de D-allulose présentant une matière sèche de 77% et une richesse en D-allulose de 95% environ.

Ces sirops sont préparés en ajustant le facteur de concentration volumique de l'étape 5 de nanofiltration de façon à obtenir différentes teneurs en Di-Allulose et/ou en préparant des sirops, en mélangeant le perméat ou le rétentat obtenu avec des cristaux de D-allulose ou de D-fructose et/ou en utilisant des membranes de nanofiltration présentant un seuil de réjection moins important. Ceci a permis d'ajuster les teneurs des différents constituants en conservant la teneur en D-allulose aux alentours de 95%. La composition de la matière sèche des sirops figure dans le Tableau 2.

Afin d'évaluer le caractère cristallisable des sirops, une amorce de cristal de D-allulose tamisé de granulométrie moyenne de 70 $\mu$m est introduite dans chacun des sirops dans une proportion de 0,3% (masse d'amorce/masse de matière sèche).

Chaque sirop ainsi amorcé est ensuite placé pendant 4 semaines dans une enceinte réfrigérée à 4°C ou à 15°C.

A l'issue de cette période, on mesure la matière sèche du surnageant (ou eaux-mères) de l'échantillon par la méthode de Karl Fischer. Plus le sirop a cristallisé, plus le surnageant présente une matière sèche basse (puisque la matière sèche du sirop s'est concentrée dans des cristaux de D-allulose).

Tableau 2 : Composition des différents sirops et matière sèche du surnageant après stockage

| Echantillo n | Composition (% CPG) | | | | | %MS du surnageant après un mois | |
|---|---|---|---|---|---|---|---|
| | D-allulose | Dimère de D-allulose | Glucose | Fructose | Autres | 15°C | 4°C |
| 1 | 95,1 | 0,1 | 1 | 3,5 | 0,3 | 70,6 | 67,5 |
| 2 | 95 | 0,5 | 0,3 | 3,5 | 0,7 | 71,8 | 68,7 |
| 3 | 94,9 | 0,7 | 0,1 | 3,2 | 1,1 | 72,8 | 69,7 |
| 4 | 95 | 1,2 | 0,2 | 1,9 | 1,7 | 73,4 | 70,2 |
| 5 | 95 | 1,5 | 0,2 | 2 | 1,3 | 73,9 | 70,8 |
| 6 | 95,2 | 2,1 | 0,2 | 1,8 | 0,7 | 74,7 | 71,3 |
| 7 | 95 | 2,7 | 0,2 | 1,4 | 0,7 | 75,3 | 71,6 |
| 8 | 95 | 3 | 0,2 | 4,1 | 0,7 | 75,3 | 71,5 |
| 9 | 95,1 | 3,4 | 0,1 | 1 | 0,4 | 75,6 | 71,6 |
| 10 | 94,9 | 4 | 0 | 1 | 0,1 | 75,3 | 71,5 |
| 11 | 94,8 | 4,6 | 0 | 0,5 | 0,1 | 75,7 | 71,8 |
| 12 | 94,9 | 5 | 0 | 0,1 | 0 | 75,2 | 71,9 |

Ces résultats se trouvent représentés à la figure 8.

Ces essais démontrent que les dimères de D-allulose sont des composés tout à fait particuliers, qui ont une influence considérable sur le caractère cristallisable d'un sirop de D-allulose le comprenant, contrairement par exemple au glucose ou au fructose.

Ainsi, plus la quantité de dimère D-allulose dans le sirop est faible, plus le sirop de D-allulose est cristallisable, ceci alors même que la quantité de D-allulose reste similaire.

## Exemple 3. Fabrication de caramels à texture longue

Une composition de caramels à texture longue (caramels durs) est réalisée selon la recette du Tableau 3 à partir du sirop de D-allulose selon l'invention (échantillon 2).

Tableau 3. Formulation de caramel

| Constituants | Proportions |
|---|---|
| | |
| Crème fraiche | 27,00 |
| Lait écrémé en poudre | 8,00 |
| Sirop d'allulose | 52,00 |
| Beurre | 5,60 |
| Sel | 0,10 |
| Eau | 6,10 |
| Lécithine | 0,20 |
| Vanille | 1,00 |
| Total | 100,00 |
| | |

(suite)

| Données nutritionnelles [a] pour une part de 30 g: | |
|---|---|
| | |
| Calories (kcal) | 99,90 |
| Total carbohydrates (g) | 18,82 |
| Sucres (g) | 18,51 |
| Protéines (g) | 0,50 |
| Matières grasses (g) | 6,68 |
| Texture | long (7-9) |
| [a] Données nutritionnelles calculées selon les spécifications des produits ou, à partir de la base de données Nutritional USDA's National Nutrient Database for Standard Reference Release 27, lorsque ces spécifications ne sont pas disponibles | |

**Conclusions:**

Un caramel de texture longue a pu être obtenu, tout en présentant l'avantage de présenter une teneur réduite en calories.

**Exemple 4. Fabrication de chewing-gums**

Une composition de chewing-gum est réalisée selon la recette ci-dessous à partir du sirop de D-allulose selon l'invention (échantillon 2).

| | |
|---|---|
| Gomme base (1) | 28,0% |
| Poudre d'allulose | 58,0% |
| Sirop d'allulose | 3,5% |
| Poudre de mannitol (diamètre moyen : 160 $\mu$m) (2) | 3,7% |
| Poudre de mannitol (diamètre moyen : 50 $\mu$m) (3) | 0,1% |
| Sirop de maltitol (4) dilué à 60% de matière sèche | 3,5% |
| Edulcorants intenses (5) | 0,2% |
| Parfums | 3,0% |

(1) Optima®, CAFOSA
(2) Pearlitol® 160C, ROQUETTE
(3) Pearlitol® 50 C, ROQUETTE
(4) Lycasin® 80/55, ROQUETTE
(5) GumSweet®, SWEETENER SOLUTIONS

La gomme base, les poudres d'allulose et de mannitol sont placées dans une enceinte à 55°C pendant 4 heures. La gomme base, 60% de la poudre d'allulose, la poudre de mannitol et la moitié du sirop de maltitol sont mélangés dans un mélangeur à gomme à 50°C. Puis, les édulcorants intenses, le reste de la poudre d'allulose, le reste du sirop de maltitol, le sirop d'allulose et les parfums sont ajoutés successivement. Le mélangeur est arrêté et la composition obtenue retirée, puis mise sous forme des tablettes de chewing-gum.

La tablette de chewing-gum présente l'avantage de présenter une bonne texture au moment de la mâche, ce qui s'explique par une dureté élevée.

**Revendications**

**1.** Sirop de D-allulose comprenant, en plus du D-allulose, une teneur massique en dimère de D-allulose exprimée en masse sèche, déterminée par chromatographie phase gaz (CPG), inférieure à 1,5%.

2.  Sirop de D-allulose selon la revendication 1 **caractérisé en ce qu'**il présente une teneur massique en dimère de D-allulose exprimée en masse sèche allant de 0,1 à 1,4%, avantageusement allant de 0,2 à 1,3%, préférentiellement allant de 0,3 à 1,2%.

3.  Sirop de D-allulose selon l'une des revendications 1 ou 2 comprenant une teneur en D-allulose supérieure ou égale à 75%.

4.  Sirop de D-allulose selon la revendication 3 **caractérisé en ce qu'**il comprend, par rapport à sa masse sèche :

    • de 75 à 99% de D-allulose ;
    • de 0 à 25% de D-fructose ;
    • de 0 à 10% de glucose ;
    • de 0 à 1,5 % de dimère de D-allulose, par exemple allant de 0,1 à 1,4%, avantageusement allant de 0,2 à 1,3%, préférentiellement allant de 0,3 à 1,2%.

5.  Sirop de D-allulose selon l'une des revendications précédentes **caractérisé en ce qu'**il présente une matière sèche supérieure à 50%, par exemple allant de 65 à 85%, notamment allant de 70 à 83%, par exemple de 75 à 82%.

6.  Procédé de fabrication d'un sirop de D-allulose selon l'une des revendications précédentes **caractérisé en ce qu'**il comprend :

    • une étape de fourniture d'une composition de D-allulose comprenant des dimères de D-allulose ;
    • une étape de nanofiltration de ladite composition de D-allulose pour fournir un rétentat et un perméat ;
    • une étape de récupération du perméat de nanofiltration ;
    • une étape de concentration de ce perméat pour fournir le sirop de D-allulose selon l'une des revendications précédentes.

7.  Procédé selon la revendication 6 **caractérisé en ce que**, pour fournir la composition de D-allulose, est réalisée une étape de chromatographie d'une composition comprenant du D-allulose et du D-fructose.

8.  Procédé selon la revendication 7 **caractérisé en ce que** la composition comprenant du D-allulose et du D-fructose est obtenue par épimérisation d'une solution de D-fructose.

9.  Sirop de D-allulose susceptible d'être obtenu par le procédé selon l'une des revendications 6 à 8.

Solution de D-fructose

*Flux 1*

Etape 1 : Epimérisation

*Flux 2*

Etape 2 : Microfiltration ⟶ Flux 8

*Flux 3*

Etape 3 : Déminéralisation

*Flux 4*

*Flux 10* ⟵ Etape 4 : Chromatographie

*Flux 5*

Etape 5 : Nanofiltration ⟶ Flux 9

*Flux 6*

Etape 6 : Evaporation

*Flux 7*

Sirop cristallisable

**Figure 1**

Solution de D-fructose

*Flux 1*

*Flux 1'*

Etape 1 : Epimérisation

*Flux 2*

Etape 2 : Microfiltration ——————→ Flux 8

*Flux 3*

Etape 3 : Déminéralisation

*Flux 4*

*Flux 12*

*Flux 4'*

*Flux 11* ← ———— Etape 4 : Chromatographie

*Flux 10*  *Flux 5*  *Flux 9*

Etape 5 : Nanofiltration

*Flux 6*

Etape 6 : Evaporation

*Flux 7*

Sirop cristallisable

**Figure 2**

**Débit l/m²/H**

Figure 3

Figure 4

Figure 5

Figure 6

Figure 7

Figure 8

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2015032761 A1
- JP 2001354690 A
- WO 2015094342 A
- WO 2016135458 A
- WO 2015032761 A
- WO 2011119004 A2
- WO 2016064087 A
- CN 104447888 A
- CN 103333935 A